# EUROPEAN PATENT APPLICATION

(11) **EP 1 666 046 A1**
(43) Date of publication of application: **07.06.2006**
(21) Application number: 03750245.7
(22) Date of filing: 17.09.2003
(51) Int. Cl.: A61K 31/7008, A61P 35/00

(54) **USE OF N-ACETYL-D-AMINOGLYCOSAMINE IN PREPARATION OF DRUGS FOR THE TREATMENT OF CACER AND METASTASIS**

(71) Applicant: Third Military Medical University Chinese People's Liberation Army P.R. of China, Chongqing 400038 (CN); Bio-Wave Institute of Suzhou Hi-Tech New District Corporation, Ltd, Jiangsu 215011 (CN); Beijing Sino-Hongkong Dafu Science & Technology of Biowave Co., Ltd., Beijing 101200 (CN)
(72) Inventor: XU, Qiwang, Shapingba District, Chongqing 400038 (CN); LIU, Junkang, Shapingba District, Chongqing 400038 (CN); YUAN, Zetao, Shapingba District, Chongqing 400038 (CN)
(74) Representative: Chung, Hsu Min
(86) International application number: PCT/CN2003/000783
(87) International publication number: WO 2005/025581

(57) **Abstract**

The present invention has disclosed a use of N-acetyl-D-glucosamine in the preparation of a medicament for treating tumors and metastatic diseases. N-acetyl-D-glucosamine is able to regulate cellular membrane, constitute a stable structural component of cellular membrane, participate metabolism process of tumor cells, and stop energy supply to cause the death of tumor cells. As a drug for adjustment therapy, it showed significant effects in the laboratory researches, animal tests and human body trials.

## Description

### Technical field

The present invention relates to the use of N-acetyl-D-glucosamine and pharmaceutical acceptable salts thereof in the manufacture of medicaments for anti-tumors and anti-metastasis.

### Background Art

Tumors are common and frequently-occurring diseases, wherein malignant tumors are the ones of severest diseases harmful to human health. According to statistics, about 5 millions die with cancers in the world per year and about 900 thousands die with cancers in China. With the development of population aging, the increase of urban population proportion and the aggravation of environmental pollution in China, the incidence rate of cancers increases daily. Cancers are usually caused by the interaction of various factors such as environment, nutrition and diet, genetic factors, viral infections and the selection of life styles.

Tumors are usually treated by various methods, such as operation, radioactive ray, anticancer drugs, traditional Chinese medicine, and immune therapy, etc., wherein operation therapy is still the most effective method. Chemical therapy develops fast, and many anticancer drugs, such as cytotoxin drugs, antimetabolite drugs, antibiotics, alkaloids, hormones, etc., have been used at present. However, the present anticancer drugs cannot solve two problems: (1) one is the therapeutic effect, chemotherapeutics merely kill tumor cells in a certain percentage, for example, if a patient of advanced leukemia with 10¹² tumor cells is treated by a chemotherapeutic that kills 99.99% tumor cells, the residual 10⁸ tumor cells still may result in clinical relapse; and (2) the other is the effect of anticancer drugs on normal cells, in particular normal cells in stage of growth and proliferation, so various adverse effects may appear after administration of such drugs, and frequent adverse effects include: (a) hypolekocytosis and platelet-reduction; (b) digestive tract reactions, such as nausea, vomiting, diarrhea, dental ulcer, etc.; (c) falling off of hair; (d) blood urine; (e) hypo-immunity; and (f) complicated bacterial or fungal infections. Other therapeutic methods, such as radiotherapy, traditional Chinese medicines and immune therapy, etc. also have similar problems. Thus, in the development of new anticancer drugs, not only the effect of killing tumor cells, but also the decrease of adverse effects should be considered.

In the research of "bio-waves" theory, the present inventor has set up a bacterial wave growth model. Through research, it is known that this wave is of its intrinsic regulation mechanism: some chemical substances are able to participate the regulation in the bio-wave process, so as to transform an abnormal periodic slow wave into a normal physiological chaotic quick wave, and these kinds of substances are known as promoting wave factors. Through separating, purifying and identifying, it is determined that one of the factors is N-acetyl-D-glucosamine, the promoting wave function of which is shown in regulating the coupled oscillation of cellular membrane proteins and glycolyx. Many biochemical and physiological processes of human body need the participation of the promoting wave factors, and it would lead to an abnormal state, if this kind of promoting wave factors is missing in the living body.

N-acetyl-D-glucosamine is a chemical reagent. From the 1990's, it is continually used to treat periodontitis (WO9102530A1), microbiological infection (WO9718790A3), intestinal inflammation (WO9953929A1), cornea disease (JP10287570A2), hypertrophy of the prostate (US05116615) and so on. It is also applied in cosmetology (JP59013708A2), shampoo preparation (JP2011505A2), tissue growth regulation agent (WO/A 8702244), and etc., but it has not been used in the manufacture of a medicament for regulating microecological balance of mucocutaneous membrane up to now.

### Contents of the invention

The inventor of the present invention surprisingly finds that N-acetyl-D-glucosamine is able to regulate cellular membrane, constitute a stable structural component of cellular membrane, participate metabolism process of tumor cells, and stop energy supply to cause the death of tumor cells. As a drug for adjustment therapy, it showed significant effects in the laboratory researches, animal tests and human body trials.

The present invention is related to the use of N-acetyl-D-glucosamine and pharmaceutical acceptable salt thereof in the manufacture of a medicament for treating tumors and metastatic diseases.

In addition, the present invention is related to a method for preventing or treating tumors and metastatic diseases, including administrating to a patient who is in need thereof an effective amount of N-acetyl-D-glucosamine or pharmaceutical acceptable salts thereof.

The molecular formula of N-acetyl-D-glucosamine is C₈H₁₅NO₆, its structure is as follows: 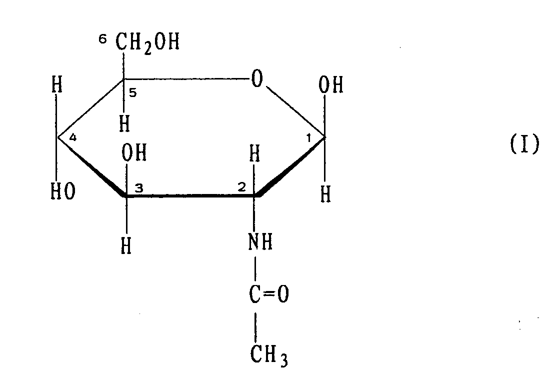 N-acetyl-D-glucosamine can be purchased in market or prepared according to known methods. For instance, patent application WO97/31121 has disclosed a method for preparing N-acetyl-D-glucosamine from chitin by enzyme method, Japanese patent application JP63273493 has disclosed a method in which chitin is partially hydrolyzed into N-acetyl-chitose, and then it is treated with enzyme to obtain N-acetyl-D-glucosamine.

The pharmaceutical acceptable salts of N-acetyl-D-glucosamine that can be mentioned are the salts formed with pharmaceutical acceptable acids, for instance, the salts formed with inorganic acids, such as hydrochloride, hydrobromide, borate, phosphate, sulfate, bisulfate and hydrophosphate, and the salts formed with organic acids, such as citrate, benzoate, ascorbate, methyl sulfate, naphthalene-2-sulfonate, picrate, fumarate, maleate, malonate, oxalate, succinate, acetate, tartrate, mesylate, tosylate, isethionate, α-ketoglutarate, α-glyceryl phosphate and glucose-1-phosphate.

Generally, the compound of the present invention is formulated for oral administration, parenteral administration, sublingual administration or transdermal administration, preferably parenteral or oral administration. When tumors or metastatic diseases are treated according to the method of the present invention, the amount of active component depends on characteristics and severity of said diseases and body weight of patient. As for an adult patient, the proposed injection dose of N-acetyl-D-glucosamine is 100-20,000 mg/day, while the proposed oral dose of N-acetyl-D-glucosamine is 1,000-10,000 mg/day.

When the present invention is applied in pharmaceutical composition for treating the above indications via oral, sublingual, subcutaneous, intramusular, intravenous, transdermal or rectal administration, the active component can be applied to animal and human in the form of dosage unit, such as freeze-dry form or mixture formed with conventional pharmaceutically acceptable carriers. Suitable dosage unit includes oral dosage forms, such as dispersible tablet, capsule, powder, granule and solution or suspension for oral administration, dosage forms for sublingual and buccal administration, dosage forms for subcutaneous, intramusular or intravenous administration, dosage forms for topical administration, and dosage form for rectal administration, preferably preparations for parenteral and oral administration, in particular injection solutions, tablets and capsules.

Solid composition in the form of tablet are made by mixing the main active component with pharmaceutical excipient, such as gelatin, starch, lactose, magnesium stearate, talc powder, Arabic gum and etc. The tablet is able to be coated with sugar or other suitable substances, or making them possess a persistent and delayed function and continually release pre-determined amount of active component.

Preparation in the form of capsule is obtained by mixing active component with diluent, and filling the obtained mixture into soft or hard capsules.

Preparation in the form of syrup or elixir may comprise active component and sweetener that would better have no caloric, methyl p-hydroxybenzoate and propyl p-hydroxybenzoate as antiseptic agent, and fragrancing agent and suitable coloring material.

Water-dispersible powder and granular preparation may comprise active component, mixed with dispersing agent or wetting agent, or suspending agent, such as polyvinylpyrrolidone, and sweetener or taste regulating agent.

Suppository for rectum administration is prepared with an adhesive which is melted at the same temperature as that of rectum, such as cocoa oil or polyethylene glycol.

The injectable aseptic aqueous solution, salt solution, alcoholic solution or homogenous suspension of the compound of the present invention can be administrated parenterally.

### Optimal embodiment for carrying out the invention

The following experimental examples are used to illustrate that the compound of the present invention (the compound of formula (I)) is capable of inhibiting the growth and development of tumors of animal in vivo.

### I. Promoting wave test of the compound of formula (I)

1. Experimental materials and method:
1.1 Samples: pure compound of formula (I)
1.2 Experimental materials:
   Strain: Proteus Mirabilis (which should comply with the following biological reaction characteristics: dynamics (+), urease (+), lactose (-), glucose (+), H₂S (-), phenylalanine deaminase (+).
   Culture medium: modified LB culture medium (the component of the composition are: 1% trytones, 0.5% yeast extract, 1% sodium chloride, 0.1% glucose, 0.002% TTC, and pH = 7.2~7.4).
1.3 Experimental method:
   The Proteus Mirabilis were inoculated at the center of a LB plate, incubated at 37°C and cultured for 9 hours, then concentric rings emerged, extended outward continually with an interval of 3 hours, and this was taken as a control; the compound of formula (I) with final concentration of 0.5 % was added onto a LB plate, and the Proteus Mirabilis were inoculated by the same method and cultured at 37°C, and the results showed that not only the concentric rings emerged with an interval of 3 hours, but also many fine waves on each ring emerged in comparison with the control.
2. Experimental results and evaluation:
   The experiment adopted a bio-wave model for studying the promoting wave function of the compound of formula (I). It can be seen from the results that the compound of formula (I) was not only able to make bacterial cell reveal a normal bio-wave characteristic, but also to cause the wave to reveal a finer wave mode and shorter wave cycle, and these indicated that the compound of formula (I) has promoting function to bio-waves, and the promoting wave function is able to regulate the cellular redistribution in vivo and is the basis that the compound of formula (I) is used as an regulator for preventing and treating microecological imbalance of mucocutaneous membrane.

### II. Toxicological test of the compound of formula (I), including:

1. Acute toxicity test : including tests of administrating medicine orally, intravenous injection and maximum limit amount for administration;
2. Ames test;
3. Micronucleus test of bone marrow cell of small mouse;
4. Abnormal sexual test for the sperm of mouse;
5. Abnormal aberrance test for the chromosin of mouse' s testis;
6. Chronic lethal test;
7. Subchronic toxicity ( feed for 90 days) test;
8. Traditional deformity-inducing test;
The results from these tests showed that in the acute toxicity test of the compound of formula (I), a dosage of more than 2g/kg was taken, which was 300 times greater than the injection dosage for human being, but the acute toxicosis reaction had not appeared. In the long-period toxicity test, the maximum dosage had reached up to 1g/kg, and after the treatment and observation for four weeks, there was no intoxication reaction yet; and in the reproduction test, the mouse was feed from routine dosage of 7mg/kg for 3 generations, and it has been proved that the compound of formula (I) had no influence on the pregnancy, birth, nursing and the growth of baby mouse. So it was proved that the compound of formula (I) was a substance without toxicity.

### III. Animal tests

1. Materials and methods
1.1 Animals: Kunming mice, Scad male mice
1.2 Tumor cells: B16 cells, PG cells and Biu cells, which were presented by from the Staff Room of Pathology of the present university and the Tumor Department of the Second Affiliated Hospital, respectively.
1.3 Methods: soles of Kunming mice and Balbc mice were injected with B 16 cell suspension, and these mice were intraperitoneally injected with 10% aqueous solution of the compound of formula (I) at 0^{th}, 4^{th} and 7^{th} day after B16 cells were injected. The results are shown in the following table.

| Group Diameter of tumor Observation time | Group I (Administered immediately) X | Group II (Administered at the 4^{th} day) X | Group III (Administered at the 7^{th} day) X | Control X |
|---|---|---|---|---|
| 3 | 0 | 0.05 | 0.1 | 0.1 |
| 7 | 0.11 | 0.12 | 0.21 | 0.25 |
| 14 | 0.18 | 0.43 | 0.60 | 0.78 |
| 21 | 0.21 | 0.58 | 1.10 | 1.32 |
| 28 | 0.15 | 0.72 | 1.38 | 1.47 |

According to the table, the growth of tumor was inhibited in the groups wherein a high concentration of compound was intraperitoneally injected; and according to the results of 3 groups, the early the compound was administered, the better the effects were. This was the basis for regulating the tumor growth, and controlling growth and metabasis of tumor. Similar results were also obtained by using PG cells and Biu cells and in the early control tests conducted in scad mice.

### IV. Typical cases

Hu, 7 years old, who was in the Department of Peadiatrics of Xinan Hospital for suffering with Hodgkin's disease, appeared severe adverse reactions during the period of chemotherapy, such as nausea, emesis, anorexia and frequent infections, When wave-promoting N-acetyl-D-glucosamine capsule was used for controlling adverse reactions, it was observed that, by continuously administering said capsules after the end of chemotherapy, the hemogram was improved, the lymphocyte bone marrow image obviously aged, the M protein level in blood decreased, and the symptoms were effectively controlled.

Chen, female, was accepted by the Department of Surgery of Chuanxinan Mining Area Hospital for suffering with breast cancer. The chief physician, Chen Qi, considered the high metastasis rate after operation, administrated Chen via injected with the compound of formula (I) in a dose of 15 g/60kg/day before the operation and for a continuous week after the operation. The result showed that no metastasis occurred in this patient within one year after the operation.

The present invention develops a new medical use of N-acetyl-D-glucosamine, expands the scope of using N-acetyl-D-glucosamine, and increases the exploitation value of N-acetyl-D-glucosamine.

## Claims

1. A use of N-acetyl-D-glucosamine in the manufacture of a medicament for treating tumors and metastatic diseases.

2. The use according to claim 1, wherein said medicament is a pharmaceutical preparation in the form of injection solution, tablet or capsule.

3. The use according to claim 1 or 2, wherein said medicament has an injection dose of 100~20,000mg/day N-acetyl-D-glucosamine, or an oral dose of 1,000~10,000mg/day N-acetyl-D-glucosamine.

4. The use according to claim 1 or 3, wherein the concentration of N-acetyl-D-glucosamine in said medicament is 0.1 ~ 10 % by weight.
